# EUROPEAN PATENT SPECIFICATION

(11) **EP 1 235 615 B1**
(45) Date of publication and mention of the grant of the patent: **15.02.2006**
(21) Application number: 01958275.8
(22) Date of filing: 23.08.2001
(51) Int. Cl.: A61N 1/04

(54) **BIOMEDICAL ELECTRODES AND BIOMEDICAL ELECTRODES FOR ELECTROSTIMULATION**
BIOMEDIZINISCHE ELEKTRODEN UND BIOMEDIZINISCHE ELEKTRODEN ZUR ELEKTRISCHEN STIMULATION
ELECTRODES BIOMEDICALES ET ELECTRODES BIOMEDICALES POUR ELECTROSTIMULATION

(30) Priority: 24.08.2000 GB 0020936; 13.10.2000 IE 000829
(43) Date of publication of application: 04.09.2002
(73) Proprietor: Unomedical Limited, Redditch, Worcestershire B98 9N1 (GB)
(72) Inventor: MCADAMS, Eric, Thomas, County Antrim BT38 9PZ (GB); ANDREWS, Philip, Newbury, Berkshire RG20 8BU (GB)
(74) Representative: Nielsen, Henrik Sten
(86) International application number: PCT/IB2001/001520
(87) International publication number: WO 2002/015974

(56) References cited:
- FR-A- 2 785 544
- US-A- 4 736 752
- US-A- 5 836 942
- US-A- 6 007 532

## Description

The present invention relates to biomedical electrostimulation electrodes, that is to say, surface electrodes for applying therapeutic electrical impulses to a patient.

In order to apply effective therapeutic electrical impulses to a patient, a suitable stimulator is connected electrically to at least one pair of electrodes attached to the patient's skin at locations judged by the therapist to be optimal for achieving the desired effect.

The distribution of current under the electrode is an important factor. In the simplest case, current density (the amount of current per unit of conduction area) is inversely proportional to the electrode/skin contact area. If the same current is applied through a pair of large electrodes and a pair of small electrodes, the stimulation effect will be most pronounced under the small area electrodes due to the increased current density. If a small electrode is used in conjunction with a large area electrode, the effect is more pronounced under the smaller of the two. In this case, the small electrode is often used as the "active" electrode to target a small area such as a motor point. The large electrode is simply used to complete the electrical circuit and is termed the "indifferent" or "dispersive" electrode.

Biomedical electrodes generally comprise a backing member (typically in sheet form), a layer of an electrically conductive hydrogel (or other electrically conductive adhesive) provided between the backing member and the patient's skin, and an electrical terminal arrangement in electrical contact with the hydrogel layer and adapted for connection to an electrical lead or apparatus. The electrode may, but need not, include a current distribution member in electrical contact between the hydrogel and the electrical terminal arrangement, which in use assists in distributing the electrical current to as wide an area of the hydrogel layer as possible.

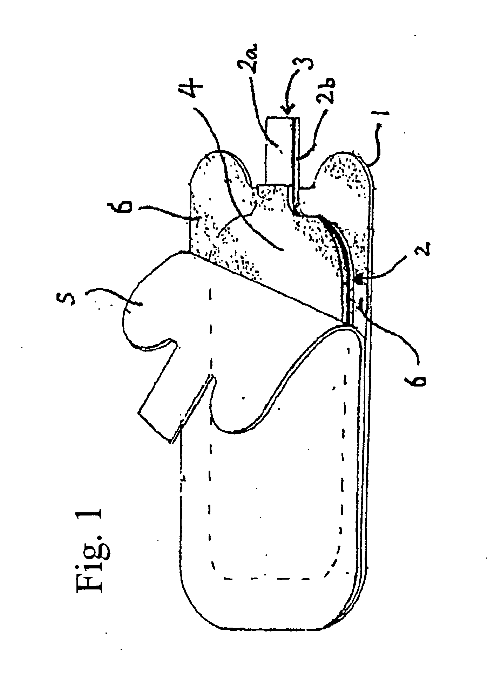

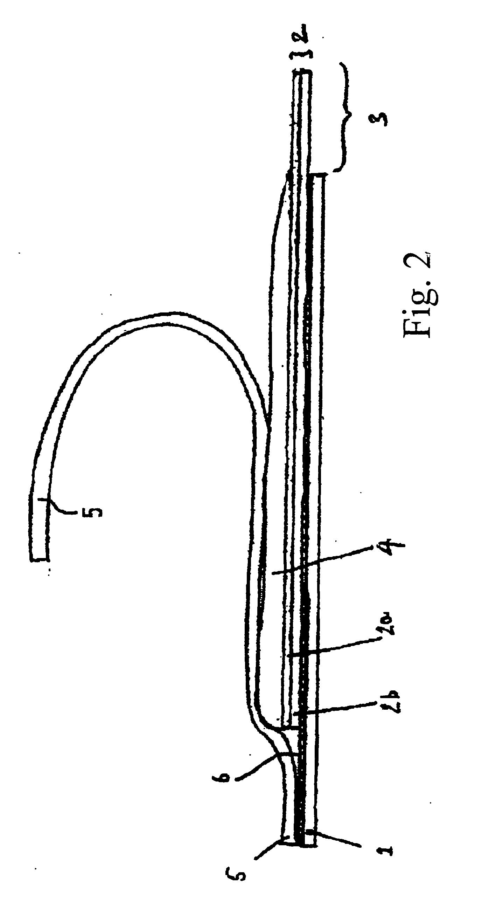

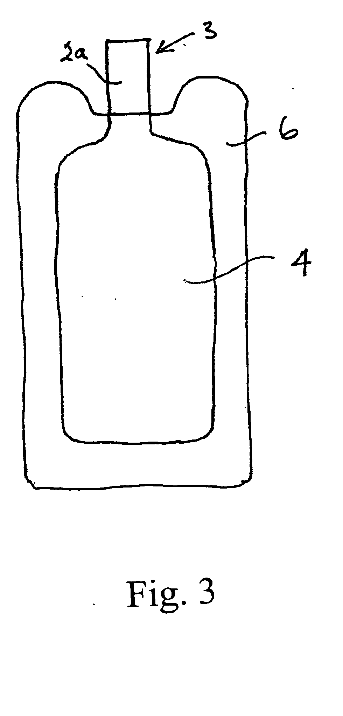

Figs. 1 to 3 of the accompanying drawings shows one known design of biomedical electrode, marketed under the name NEUTRALECT (TM) (MSB Limited, Ramsbury, UK, tel +44 1672 522 100). This prior art electrode is used as an electrosurgical dispersive electrode, and is typically affixed to the patient's skin during electrosurgery to enable an electrical circuit to be completed through the patient's body. The electrode is quite large, over 15cm in length. As shown in the figures, an electrically insulating backing member 1 in the form of a sheet of polyester foam has a current distribution member 2 stuck to it by a layer of nonconductive pressure-sensitive adhesive 6. The member 2 is a bilayer of an aluminium foil layer 2a on and coextensive with a non-foam polyester support layer 2b. The current distribution member 2 is of smaller dimensions than the adhesive-covered foam layer 1 such that latter extends beyond the periphery of the member 2 to provide secure adhesion of the electrode to the skin, even in the presence of fluids, and to reduce the risk of a member of the operating team accidentally touching the foil layer 2a when it is live. The purpose of the non-foam layer 2b is to support the relatively fragile foil layer 2a during manufacture. An electrical terminal comprises a tab formation 3 which is an integral lateral extension of the current distribution member 2 and is adapted to be gripped by electrically conductive jaws of a crocodile clip or the like (not shown) connected to a current supply lead.

The electrode also includes a layer 4 of an electrically conductive adhesive hydrogel layer, which in use adheres to the patient's skin and serves as an electrical connection between the patent and the foil layer 2a. With the exception of the terminal 3, which is not covered by the layer 4, the hydrogel layer 4 completely covers the foil layer 2a, sandwiching the foil layer 2a between it and the backing member 1. The hydrogel layer 4 is protected before use by a release layer 5 of siliconised paper or the like, which as shown is simply peeled off before use. Although electrically conductive, the hydrogel layer 4 has a substantially greater resistivity than the foil layer 2a.

Under a given electrode, it is generally highly desirable to have a uniform current density, since an increased concentration of the current at one or more points ("hot spots") under the electrode may cause electrode deterioration or pain and trauma to the patient at otherwise safe overall current densities. At best in such cases, the applied current may have to be limited to less than therapeutic values due to the patient's discomfort.

With the highly conductive metal electrodes used for external cardiac pacing, defibrillation and electrosurgery, current density hot spots are often observed to occur under the perimeter of the electrode.

It has been shown (Wiley and Webster, "Analysis and Control of the Current Distribution under Circular Dispersive Electrodes", IEEE Trans. Biomed. Eng., Vol. BME-29, pp. 381-385, 1982) that up to half of the total current flowing through a circular electrode can flow through an annular perimeter region having a width about 1/7th of the radius of the electrode. This is referred to in the literature as the fringe, edge or perimeter effect, and helps explain the observation that trauma due to current density hot spots often occurs on the skin underlying the peripheral edge of an electrostimulation electrode.

A simple intuitive picture will aid the understanding of this effect. When electrical current flows into a highly conductive metal electrode, for example the foil layer 2a of Fig. 1, it has the choice of continuing to flow through the conductive metal foil or into the more resistive hydrogel-skin interface. The electrical current chooses the path of least resistance and much of it continues to flow through the metal until it reaches the perimeter of the plate where it then is forced to flow through the gel into the patient.

Many suggestions have been made to reduce this edge effect observed with metal electrodes. These include:
(1) increasing the overall area of the electrode, thus decreasing the current density, or making the gel pad slightly larger than the electrode to enable the electric field lines to spread out before reaching the skin. Although this can solve the current density 'hot spot' problem, the use of large electrodes is not cost effective and is less than optimal when used on small patients, on small or curved parts of the body and in applications were the applied stimulus has to be targeted accurately. Using a gel pad much larger than the size of the electrode has less effect than would be expected as the perimeter of such a large gel pad will carry little current.
(2) increasing the overall resistance or thickness of the gel layer in order to give the current more "time" to spread out evenly through the gel. This can also be shown to be effective. The increased resistance will however lead to energy wastage and a thicker electrode structure would not be acceptable from an aesthetic or flexibility point of view. Manufacture would probably be problematic.
(3) increasing the resistance or thickness of the gel at the edges; For example, in IEEE Trans. Biomed. Eng., Vol. BME-33, pp. 845 - 853 (1986), Kim et al proposed an annular electrode in which the resistivity of the hydrogel layer varies as a function of radial distance from the centre, for use both in low-energy and also in high-energy applications such as electrosurgery and defibrillation. Although theoretically quite attractive, this solution is not practical from a manufacturing point of view.
(4) Depositing resistive layers on the outer edge of the electrode conductive plate, thus "forcing" more current to flow through the central portion of the electrode. US Patent No. 5836942 (Netherly et al, 1998) describes a power coupling electrode in which a generally annular field of "lossy dielectric" material is applied to the periphery of the current distribution member and the whole is overlain with the hydrogel layer. The arrangement is stated to reduce the extent of peripheral hot spots. Again, this solution is not without problems from a manufacturing point of view. Moreover, creating substrate areas of preferentially low impedance in the non-peripheral region of an electrode runs the risk of causing a high current density if the electrode becomes partially lifted or dislodged with current flowing, or if the electrode is not applied properly initially.
(5) Cutting the metallic plate so that the length of the perimeter is increased and hence the peripheral current density is decreased; the metallic plate generally has the form of a 'flower' with 'petals' extending towards the perimeter of the electrode. This solution is widely used in certain therapeutic applications by several companies. However it involves a relatively complex fabrication process and results in considerable wastage of the 'stamped out' portions of the conductive layer.
(6) US Patent No. 4736752 (Munck et al, 1988) describes a power coupling electrode in which a current distribution member formed by the deposition of a layer of a conductive ink on an insulating backing layer is provided with a regular and predetermined array of voids (non-conductive areas) arranged over its surface area. The arrangement is said to control the extent of peripheral hot spots. This solution suffers from similar problems to those of solution (5). Additionally, our tests indicate that the existing embodiments of this design are ineffective at significantly reducing current flow to the periphery of the conductive layer.

It is a object of the invention to provide an improved biomedical electrostimulation electrode in which the occurrence of hot spots may be reduced or eliminated.

Accordingly, the present invention provides a biomedical electrode comprising an electrically insulating backing member, a layer of conductive foil on the backing member, the foil layer comprising a terminal for attachment of a current supply lead, and a layer of an electrically conductive adhesive on the foil layer for fixing the electrode to a patient's skin and providing a current path from the foil layer to the patient's skin, the adhesive having a greater resistivity than the foil layer, wherein the foil layer has at least one aperture stamped therein.

Preferably the aperture has a concave edge proximal to the terminal.

The aperture may be a slit or a void in the foil layer.

The invention also provides a method of manufacturing a biomedical electrode comprising an electrically Insulating backing member, a layer of conductive foil on the backing member, and a layer of electrically conductive adhesive on the foil layer, the method comprising the step of stamping an aperture through the adhesive and into the conductive foil.

The term "foil" as used herein includes discrete foil layers as well as metallic printed layers provided on an electrically insulating backing member.

Embodiments of the invention will now be described, by way of example, with reference to the accompanying drawings, in which:
Fig. 1, previously described, is a perspective view of a biomedical electrostimulation electrode according to the prior art;
Fig. 2 is a cross-sectional view, not to scale, of the electrode of Fig. 1;
Fig. 3 is a top plan view of the electrode of Fig. 1, omitting the protective liner;
Fig. 4 is a top plan view, similar to that of Fig. 3, of a biomedical electrostimulation electrode according to an embodiment of the invention;
Fig. 5 is a detailed cross-section through the electrode of Fig. 4 in the vicinity of a slit 7;
Figs. 6(a) to 6(c) show various configurations of aluminium foil layers which can be substituted for that in Fig. 4 to provide further embodiments of the invention;
Figs. 7 to 10 are a top plan views, similar to that of Fig. 3, of biomedical electrostimulation electrodes according to yet further embodiments of the invention; and
Figs. 11 to 14 show the results of tests carried out on the electrode of Fig. 8.

Although computer modeling of the current distribution under biosignal electrodes has greatly improved over recent years, only the most simplistic electrode designs can be reasonably well modeled. Unfortunately, therefore, the design of suitable electrodes remains a tedious, empirical process.

The inventors therefore developed their own simple yet effective technique of directly observing the presence of current density 'hot spots'. In their tests they used conductive silver layers formed by the deposition of silver coatings onto flexible substrates using a range of techniques including seriography. The layers were cut into various shapes, assembled as electrodes and large currents applied across the samples and an indifferent electrode which completed the circuit. As silver is changed into the relatively dark silverchloride upon the passage of current and in the presence of a chloride containing gel, the resultant current density distribution can readily be observed by inspection of the 'photograph' which forms on the conductive layer.

The inventors observed many of the anticipated effects of the shape of the conductive layer on the current density distribution. However, for conductive layers with cutout 'voids' the results were surprising. Circular voids appeared to have little effect in forcing current to enter the hydrogel layers at their "internal peripheral edges", the current appeared to largely flow around such voids and continue to the outer edges of the conductive layer.

However, concave slits facing generally towards the current supply terminal were observed to have a marked effect on current density distribution, with the concave proximal edges of the slits effectively blocking the lateral flow of current, forcing the 'trapped' current to flow into the hydrogel and thus achieving a more uniform current density distribution over the surface of the conductive layer.

Furthermore, since no material is removed when simple slits are made, the surface area of the electrode is maintained at a maximum.

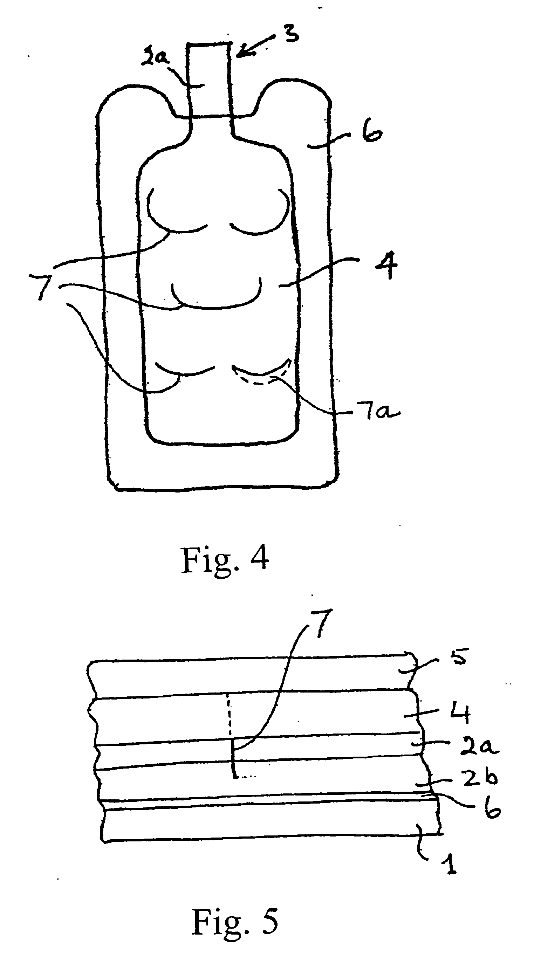

An embodiment of the invention which incorporates such concave slits is shown in Fig. 4. This embodiment is essentially the same as the known embodiment of Figs. 1 to 3, with the addition of a 2-dimensional array of concave slits 7 formed in the aluminium foil layer 2a. Each slit 7 has its concave edge proximal to the terminal 3.

The provision of multiple slits 7 at different distances from the terminal 3 enhances the effect referred to above, in that the slits 7 further away from the terminal 3 appear to 'capture' current which manages to 'slip between' the more proximal slits, much like the capture of metal balls by concave barriers in bagatelle.

The slits 7 in the foil layer 2a are formed by stamping the electrode with a suitably shaped cutting die during manufacture, after the deposition of the hydrogel layer 4 but before the application of the release layer 5. As shown in Fig. 5, this means that the gel layer 4 will be cut along with the layer 2a, but this cut will "heal" by gradual flowing together of the gel on either side of the cut (this is indicated by the dashed lines in Fig. 5). The die will also need to penetrate partially into the non-foam layer 2b to ensure complete separation of the opposite sides of the slits 7 in the foil layer 2a.

The slits could alternatively be stamped through the backing layer and into the conductive layer, or the foil could be stamped before the layers are assembled, but it has been found that the most advantageous method of creating the slits is to stamp through an adhesive layer which subsequently heals over.

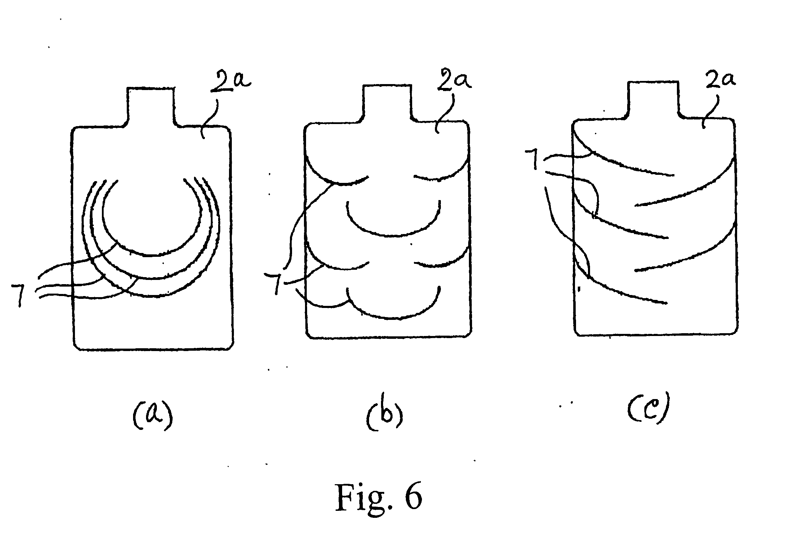

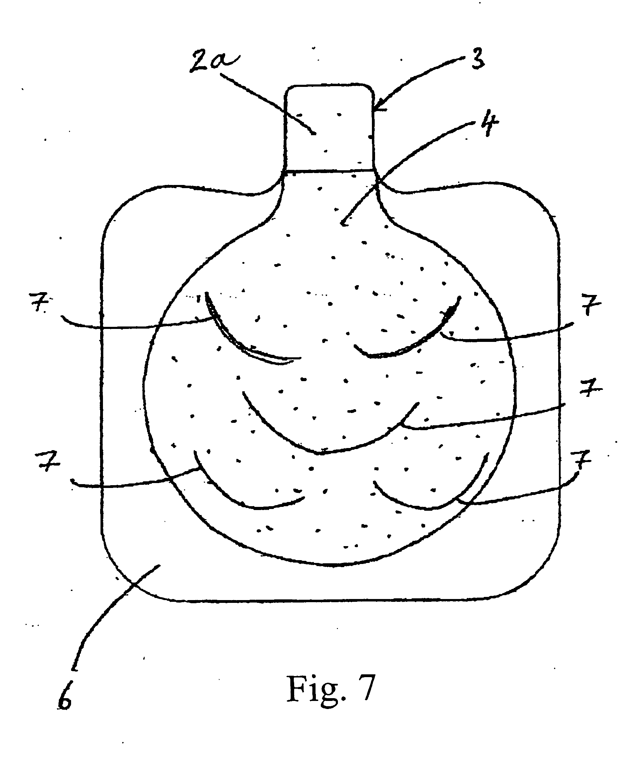

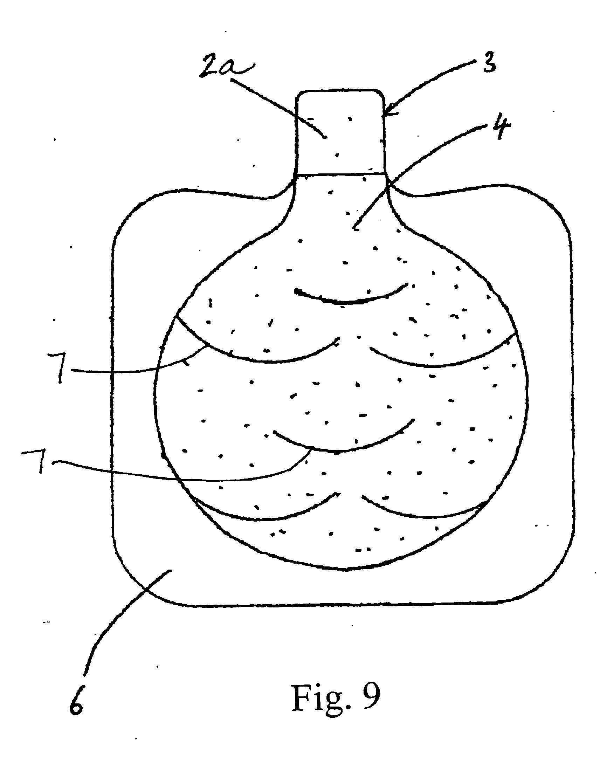

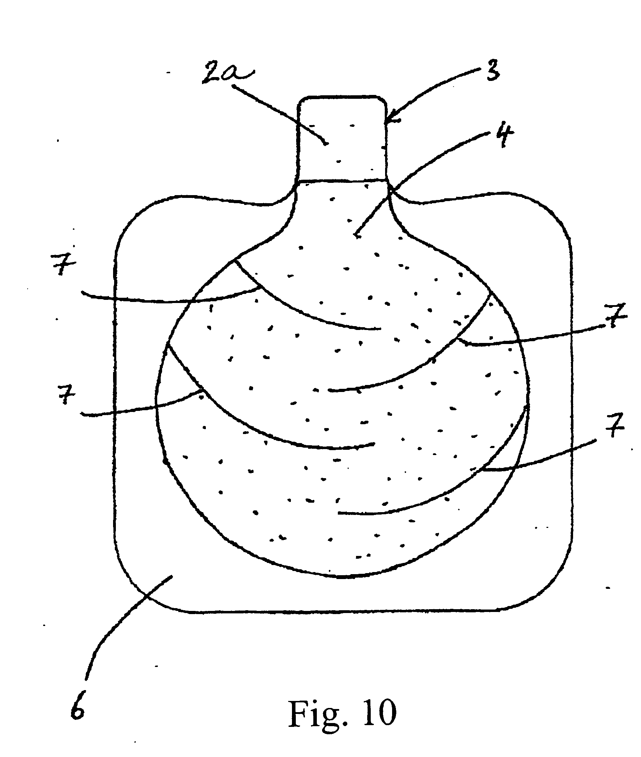

Some alternative slit layouts are shown in Figs. 6(a) to 6(c). It will be noted that the above effect is further enhanced in Figs. 6(b) and 6(c) by at least some of the slits 7 extending right to the peripheral edge of the foil layer 2a. Further embodiments of the invention which use a circular foil layer 2a, but which may in other respects be the same as the electrode of Fig. 1, are shown in Figs. 7 to 10. In all cases the slits 7 have their concave edges proximal to the terminal 3 and are stamped into the foil layer 2a in the manner described with reference to Fig. 5.

Since the "active" edge of each slit 7 is predominantly the proximal concave edge facing the terminal 3, it is possible, instead of or as well as concave slits 7, to provide voids in the foil layer 2a to serve the same purpose. Such voids will have edges which are concave proximal to the terminal 3. An example of a void which may be used instead of a slit is shown in dashed lines at 7a in Fig.4. Such voids 7a are also stamped out by a cutting die, but this occurs earlier in the manufacturing process, before the member 2 is attached to the insulating backing layer 1.

It is to be understood that the term "concave" as used herein includes any generally tray or trough-like aspect and does not require the proximal edges of the slit or void to be continuously curved.

### Experimental Results

### 1. Current distribution

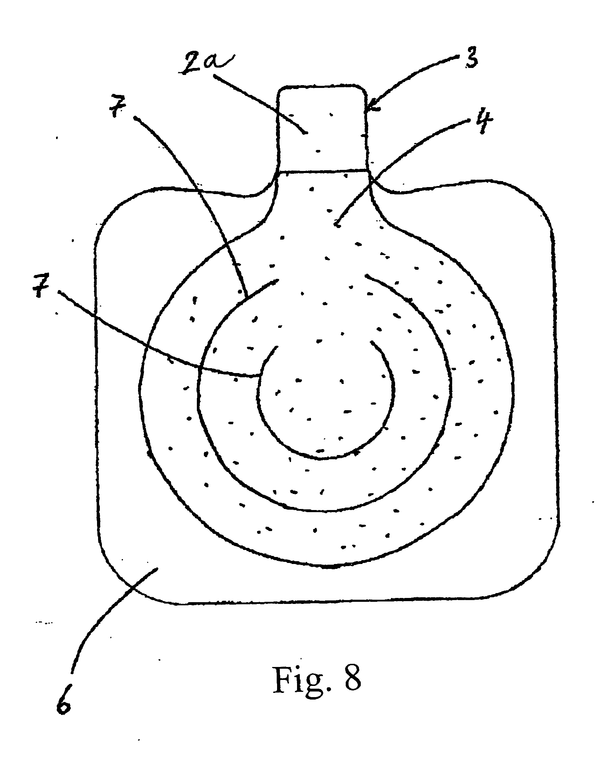

Tests were carried out by Stuckenbrock GmbH, Germany on the current distribution under electrodes of the form shown in Fig. 8, using their GP Test II apparatus. The electrode under test was applied, gel down, to a plate comprising 180 small (1 cm2) sensors arranged in a grid (8 X 10). A constant ac voltage was applied across the test electrode and the sensing plate. The frequency of the applied voltage in this instance was 500 kHz in order to harmonise with the ANSI/AAMI HF18-1993 standard for minimum safety and performance for electrosurgical systems (see below). At each sensor in the grid the conductance (reciprocal of resistance) was calculated and was proportional to the local current flowing though the test electrode in the area above a given sensor (I= V/R= VG).

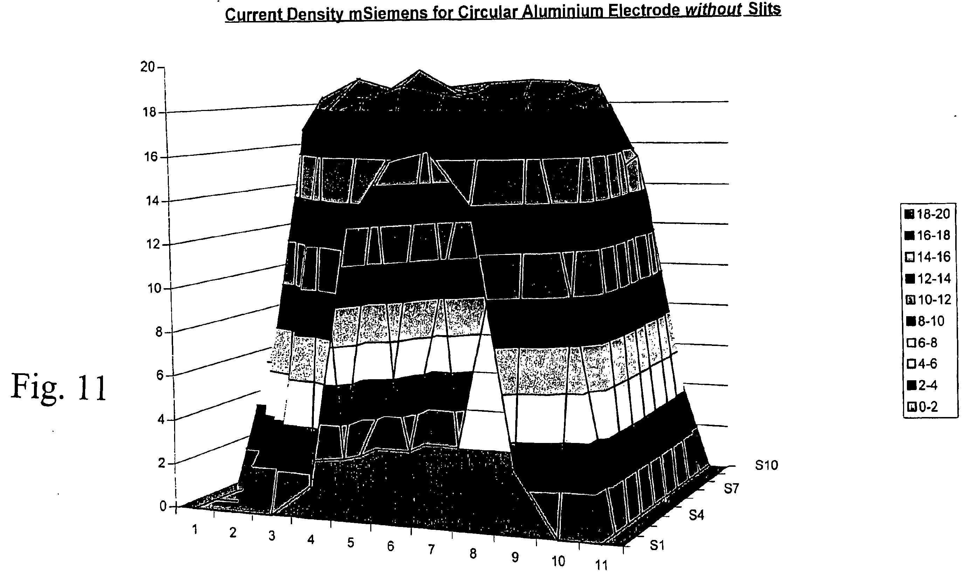

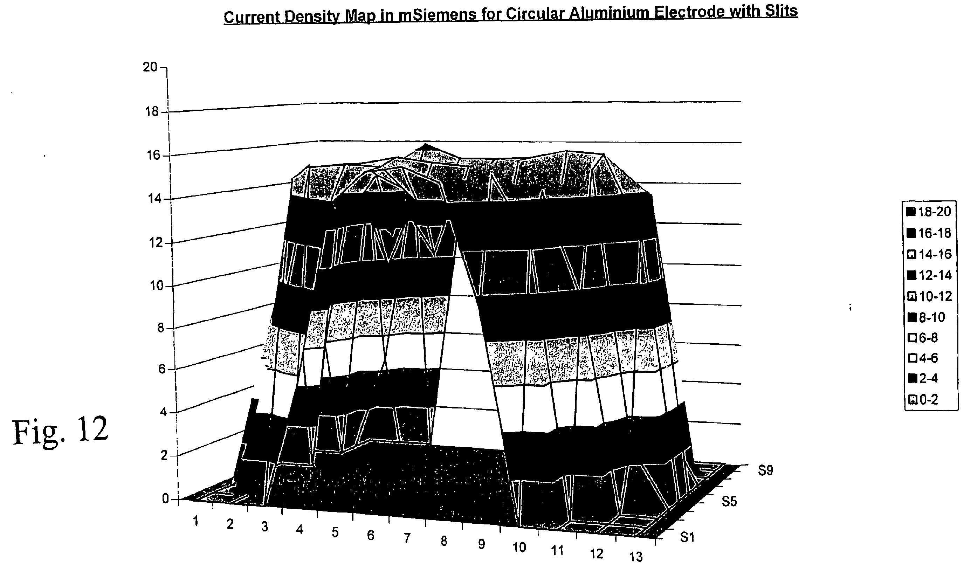

A plot of the distribution of conductance (and hence local current) under the test electrodes are shown in Figs. 11 and 12. It can be seen that the current densities under the aluminium electrode with slits (Fig. 12) is significantly less than that under the same electrode without slits (Fig. 11).

### 2. Temperature rise

As current flows through an electrode and into the patient's skin it gives rise to an increase in temperature. If too much current flows through a small area of skin it can give rise to pain and tissue trauma. In electrosurgery, for example, the ANSI/AAMI HF18-1993 standard for minimum safety and performance for electrosurgical systems requires measurement of the distribution of temperature increases under an electrode and stipulates a maximum temperature rise of not more than 6 °C following the application of a current of 700 mA 500 kHz for 60 sec.

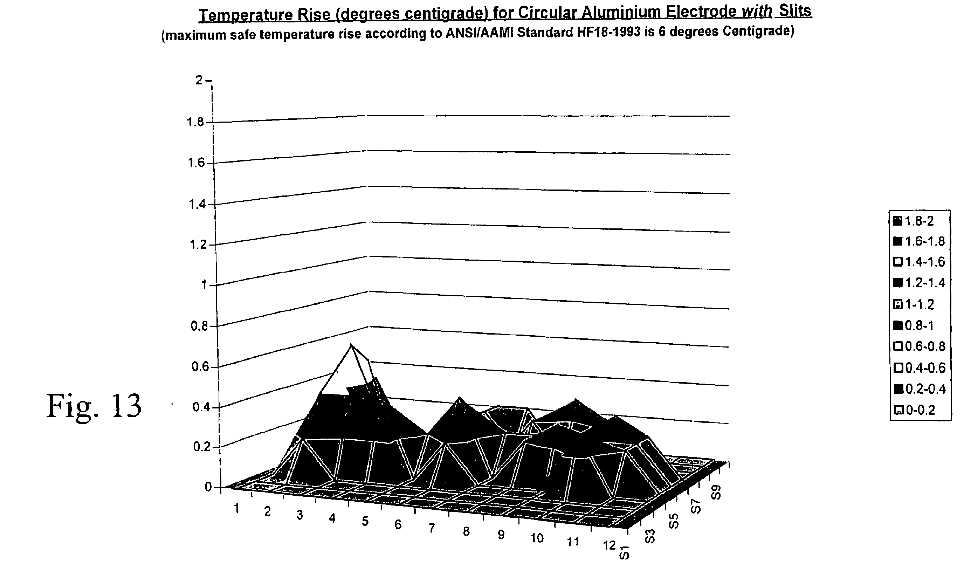

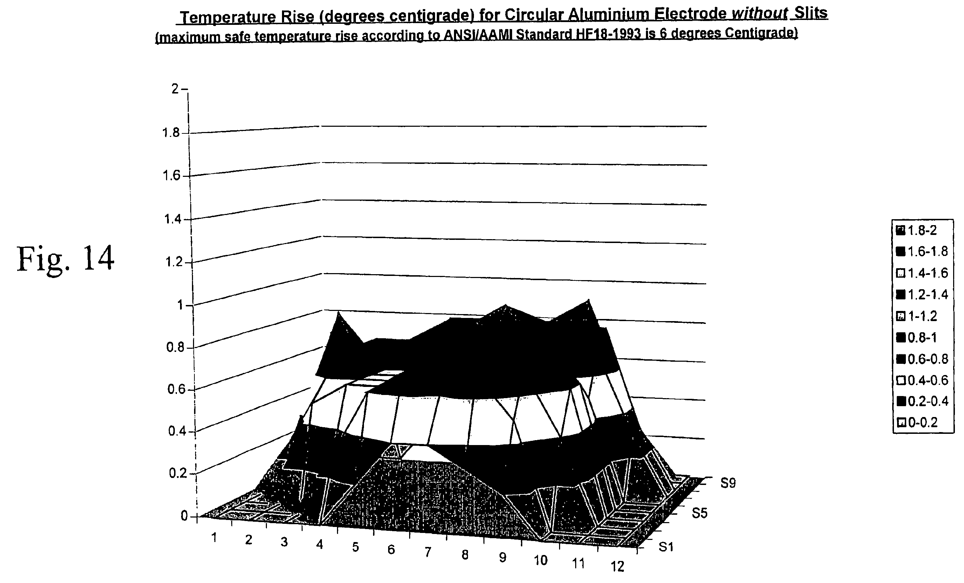

The embodiment of the invention as described above was tested by Stuckenbrock GmbH, Germany and the results in Figs. 13 and 14 were observed. The figures show temperature increase across the surface of the electrode against a scale proportionate to the 6°C Maximum allowed by the ANSI/AAMI HF18-1993 Standard. The temperature rise under the electrode with slits (Fig. 14) is relatively uniform (indicating good distribution of the current) and is significantly less than under the electrode without slits (Fig. 13). The maximum temperature rise was 0.6 °C, well within the ANSI/AAMI HF18-1993 standard.

The invention is not limited to the embodiments described herein which may be modified or varied without departing from the scope of the invention.

## Claims

1. A biomedical electrode structure adapted to contact in use an area of a patient's skin to conduct electrical current thereto or therefrom, the electrode comprising:
i) a backing member;
ii) an electrically conductive gel layer (4) for contacting the patient's skin;
iii) an electrical terminal arrangement (3) adapted for connection to an electrical lead or apparatus; and
iv) a current distribution member, comprising a relatively thin, electrically conductive layer (2a), the current distribution member contacting the gel layer (4) via an interface between (4) the conductive layer of the current distribution member and the gel layer (4) and providing an electrical connection between the gel layer (4) and the electrical terminal arrangement (3), the conductive layer (2a) of the current distribution member having at least one aperture stamped therein to substantially hinder current flow from the terminal (3) to the peripheral edges of the distribution member, **characterised in that** the aperture(s) is/are a slit (7) in the distribution member.

2. An electrode structure as in claim 1 wherein the aperture(s) has/have a concave edge proximal to the terminal to substantially hinder current flow from the terminal to the peripheral edges of the distribution member.

3. An electrode structure, as in claim 1 wherein 2-dimensional arrays of concave slits (7) are so arranged to substantially hinder current flow from the terminal (3) to the peripheral edges of the distribution member.

4. An electrode structure as in claim 3 wherein the concave slits (7) of the 2-dimensional array may extend to the lateral/peripheral edges of the distribution member to effectively create further concave internal peripheral edges and thus further hinder current flow from the terminal (3) to the peripheral edges of the distribution member.

5. An electrode structure as in claim 1 wherein arrays of concave slits (7) are so arranged as to be almost concentric.

## Patentansprüche

1. Biomedizinische Elektrodenstruktur, die dazu eingerichtet ist, beim Gebrauch einen Bereich der Haut des Patienten zu berühren, um elektrischen Strom dazu oder davon zu leiten, wobei die Elektrode umfasst:
i) ein Trägerelement;
ii) eine elektrisch leitende Gelschicht (4) zum Berühren der Haut des Patienten;
iii) eine elektrische Anschlussklemmenanordnung (3), die zum Verbinden mit einer elektrischen Zuleitung oder Vorrichtung eingerichtet ist; und
iv) ein Stromverteilungselement, das eine verhältnismäßig dünne, elektrisch leitende Schicht (2a) umfasst, wobei das Stromverteilungselement über eine Grenzfläche zwischen der leitenden Schicht des Stromverteilungselementes und der Gelschicht (4) die Gelschicht (4) berührt und einen Stromanschluss zwischen der Gelschicht (4) und der elektrischen Anschlussklemmenanordnung (3) herstellt, wobei die leitende Schicht (2a) des Stromverteilungselementes mindestens eine darin gestanzte Öffnung aufweist, um Stromfluß von der Anschlussklemme (3) zu den Umfangskanten des Verteilungselementes zu behindern, **dadurch gekennzeichnet, dass** die Öffnung(en) ein Schlitz (7) im Verteilungselement ist/sind.

2. Elektrodenstruktur nach Anspruch 1, bei der die Öffnung(en) eine konkave Kante proximal zur Anschlussklemme aufweist/aufweisen, um Stromfluß von der Anschlussklemme zu den Umfangskanten des Verteilungselementes im Wesentlichen zu behindern.

3. Elektrodenstruktur nach Anspruch 1, bei der zweidimensionale Anordnungen konkaver Schlitze (7) so angeordnet sind, dass sie Stromfluß von der Anschlussklemme zu den Umfangskanten des Verteilungselementes im Wesentlichen behindern.

4. Elektrodenstruktur nach Anspruch 3, bei der die konkaven Schlitze (7) der zweidimensionalen Anordnung zu den Seiten-/Umfangskanten des Verteitungselementes hin verlaufen können, um weitere konkave innere Umfangskanten effektiv zu schaffen und so Stromfluß von der Anschlussklemme zu den Umfangskanten des Verteilungselementes weiter zu behindern.

5. Elektrodenstruktur nach Anspruch 1, bei der Anordnungen konkaver Schlitze (7) so angeordnet sind, dass sie fast konzentrisch sind.

## Revendications

1. Une structure d'électrode biomedicale adaptée pour entrer en contact, lors de son utilisation, avec une zone de la peau d'un patient ou d'une patiente pour conduire du courant électrique à ladite zone ou de ladite zone, l'électrode comprenant:
i) un élément de support;
ii) une couche de gel électriquement conductrice (4) pour entrer en contact avec la peau du patient ou de la patiente;
iii) un dispositif de borne électrique (3) adapté pour la connexion à une conduite électrique ou un appareil électrique; and
iv) un élément de distribution de courant comprenant une couche électriquement conductrice relativement mince (2a), l'élément de distribution entrant en contact avec la couche de gel (4) à travers une surface de contact entre la couche conductrice de l'élément de distribution et la couche de gel (4) et fournissant une connexion électrique entre la couche de gel (4) et le dispositif de borne électrique (3), la couche conductrice (2a) de l'élément de distribution de courant ayant au moins une ouverture estampée là-dedans, essentiellement pour prévenir le mouvement de courant de la borne (3) aux bords périphériques de l'élément de distribution, **caractérisé en ce que** l'ouverture ou les ouvertures est/sont une fente (7) dans l'élément de distribution.

2. Une structure d'électrode ainsi que dans la revendication 1, où l'ouverture ou les ouvertures a/ont un bord concave à la proximité de la borne, essentiellement pour prévenir le mouvement de courant de la borne aux bords périphériques de l'élément de distribution.

3. Une structure d'électrode ainsi que dans la revendication 1, où les alignements à deux dimensions de fentes concaves (7) sont constitués ainsi que le mouvement de courant de la borne (3) aux bords périphériques de l'élément de distribution est essentiellement évité.

4. Une structure d'éléctrode ainsi que dans la revendication 3, où les fentes concaves (7) de l'alignement à deux dimensions peuvent s'étendre aux bords latéraux/périphériques de l'élément de distribution afin de créer de manière efficace/réelle des bords périphériques internes concaves supplémentaires et d'ainsi prévenir ultérieurement le mouvement de courant de la borne (3) aux bords périphériques de l'élément de distribution.

5. Une structure d'éléctrode ainsi que dans la revendication 1, où des allignements de fentes concaves (7) sont constitués de manière à être presque concentriques.
